(19) Europäisches Patentamt
European Patent Office
Office européen des brevets

(11) Veröffentlichungsnummer: **0 141 909**
**A2**

(12) # EUROPÄISCHE PATENTANMELDUNG

(21) Anmeldenummer: **84108792.7**

(22) Anmeldetag: **25.07.84**

(51) Int. Cl.[4]: **C 07 D 249/08**, A 01 N 43/653

(30) Priorität: **10.11.83 DE 3340989**

(43) Veröffentlichungstag der Anmeldung: **22.05.85 Patentblatt 85/21**

(84) Benannte Vertragsstaaten: **BE CH DE FR GB IT LI LU NL SE**

(71) Anmelder: **SCHERING AKTIENGESELLSCHAFT Berlin und Bergkamen, Müllerstrasse 170/178 Postfach 65 03 11, D-1000 Berlin 65 (DE)**

(72) Erfinder: **Skötsch, Carlo, Dr., Kaiserin-Augusta-Allee 83, D-1000 Berlin 10 (DE)**
Erfinder: **Krähmer, Hansjörg, Dr., Fürstendamm 10 A, D-1000 Berlin 28 (DE)**
Erfinder: **Baumert, Dietrich, Dr., Schulzendorferstrasse 108 B, D-1000 Berlin 28 (DE)**
Erfinder: **Arndt, Friedrich, Dr., Mühlenfeldstrasse 10, D-1000 Berlin 28 (DE)**

(54) **E-Triazolyl-Pentenole, Verfahren zur Herstellung dieser Verbindungen sowie diese enthaltende biozide und wuchsregulatorische Mittel.**

(57) Die Erfindung betrifft neue E-Triazolyl-pentenole der allgemeinen Formel

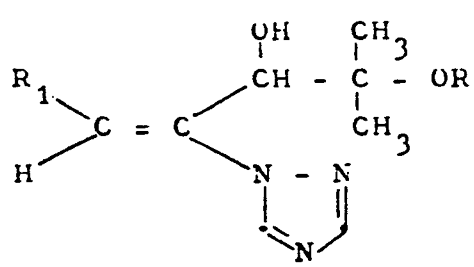

I

in der

R einen $C_1$–$C_{10}$-Alkylrest oder einen $C_3$–$C_8$-Alkenylrest und

$R_1$ einen nichtsubstituierten oder einen ein- oder mehrfach, gleich oder verschieden durch Halogen, $C_1$–$C_4$-Alkyl, $C_1$–$C_4$-Alkoxy, $C_1$–$C_4$-Alkylthio, Trifluormethyl oder die Nitrogruppe substituierten aromatischen Kohlenwasserstoffrest oder einen heterocyclischen Kohlenwasserstoffrest bedeuten und deren Säureadditionssalze mit anorganischen und organischen Säuren, Verfahren zur Herstellung dieser Verbindungen sowie diese enthaltende biozide und wuchsregulatorische Mittel.

EP 0 141 909 A2

Die Erfindung betrifft neue E-Triazolyl-pentenole gemäß Oberbegriff des Anspruchs 1, ein Verfahren gemäß Oberbegriff des Anspruchs 14 zur Herstellung dieser Verbindungen sowie Mittel gemäß Oberbegriff der Ansprüche 19 und 23 enthaltend diese Wirkstoffe.

Konstitutionsanaloge Verbindungen gleicher Wirkungsrichtung sind bereits bekannt (DE-OS 30 10 560; DE-OS 31 48 742).

Aufgabe der vorliegenden Erfindung ist die Schaffung neuer Wirkstoffe mit überlegenen Eigenschaften.

Diese Aufgabe wird erfindungsgemäß durch den in den Ansprüchen gekennzeichneten Gegenstand gelöst. Vorteilhafte Weiterbildungen sind in den Unteransprüchen beschrieben.

Die erfindungsgemäßen Verbindungen weisen überraschenderweise eine im Vergleich zu den bekannten konstitutionsanalogen Wirkstoffen überlegene biozide Wirkung und eine besonders vorteilhafte wuchsregulatorische Wirkung für Pflanzen auf und bereichern daher den Stand der Technik auf diesem Gebiet erheblich.

Die wachstumsregulatorische Wirkung entfaltete sich bei verschiedenen Pflanzenarten, wie zum Beispiel Getreidearten, Grasarten, Baumwolle, Obstbäumen und Zierpflanzen.

Bei Getreidearten wird eine Halmverkürzung erreicht, was eine größere Standfestigkeit der Pflanze zur Folge hat.

Grasarten im Rasen werden im Wuchs gehemmt, so daß Schnittarbeiten entfallen.

Bei Baumwolle bewirkt die Wuchshemmung ein gleichmäßigeres Ausreifen der Kapseln und verursacht höhere Erträge.

-2-

Bei Obstbäumen führt die gezielte Beeinflussung des vegetativen Wachstums ebenfalls zur Ertragssteigerung.

Bei Zierpflanzen hat die Wuchsreduktion den Effekt, daß mehr Pflanzen je Stellfläche angezogen werden können.

Die erfindungsgemäßen Verbindungen haben weiterhin den Vorteil, daß bei spezieller Applikation das Wachstum unerwünschter Arten, wie zum Beispiel einiger Unkräuter so gehemmt wird, daß die Kulturpflanze im Bestand einen Wachstumsvorteil erhält, mithin also der Effekt von Herbiziden erreicht wird. Mitunter sind auch die hemmenden Eigenschaften der Verbindungen zum Unterbinden der generativen Phase einsetzbar, beispielsweise bei Zuckerrüben, die nicht vorzeitig blühen sollen.

Die erfindungsgemäßen Verbindungen eignen sich überraschenderweise auch zur Erhöhung der Streßresistenz bei Kulturpflanzen wie Baumwolle, Reis, Mais, Soja, Weizen und anderen.

Besonders hervorzuheben ist die Erhöhung der Trockenresistenz

Die erfindungsgemäßen Verbindungen können sowohl im Vorauflauf- als auch im Nachauflaufverfahren angewendet werden.
Sie besitzen ein breites Wirkungsspektrum.
Die Aufwandmengen betragen zweckmäßigerweise von 0,001 bis 5 kg Wirkstoff/ha.
Die Anwendungszeit richtet sich nach dem Anwendungsziel und den klimatischen Bedingungen.

Die fungizide Wirkung der erfindungsgemäßen Verbindungen richtet sich überraschenderweise gegen Pilze unterschiedlichster systematischer Stellung. Bei Behandlung oberirdischer Pflanzenteile schützen sie gegen windbürtige Krankheitserreger. Gegen samenübertragbare Krankheitserreger kann man die Verbindungen zur Saatgutbehandlung einsetzen.



Postanschrift: Schering Aktiengesellschaft, Postfach 65 03 11, D-1000 Berlin 65 · Fur Besucher Berlin-Wedding, Mullerstraße 170-178 Telegramme: Scheringchemie
Vorstand Dr Herbert Asmis Dr Christian Bruhn Dr Heinz Hannse Horst Kramp, Dr Klaus Pohle Dr Horst Witzel · Vorsitzender des Aufsichtsrats: Hans-Jürgen Ha
Sitz der Gesellschaft Berlin und Bergkamen Handelsregister AG Charlottenburg 93 HRB 283 und AG Kamen HRB 0061 Berliner Commerzbank AG, Berlin, Kor
108700600 Bankleitzahl 100 400 00 Berliner Handels- und Frankfurter Bank, Berlin, Konto-Nr 70045224 Bankleitzahl 100 202 00 Deutsche Bank Berlin AG, Kor
2415008 Bankleitzahl 100 700 00 Postscheckamt Berlin West Konto-Nr 1175-101 Bankleitzahl 100 100 10

Außerdem wirken diese systemisch, das heißt sie werden von der Wurzel der Pflanzen zum Beispiel nach Einbringen bei der Saat aufgenommen, werden in die oberirdischen Teile der Pflanze tranportiert und schützen diese gegen Krankheitserreger.

Die erfindungsgemäßen Verbindungen weisen außerdem eine starke bakterizide Wirkung auf.

Wegen des erkannten breiten Wirkungsspektrums eignen sich die Verbindungen nicht nur zum Schutz von Kulturpflanzen sondern auch zum Materialschutz und zur Bekämpfung humanpathogener und tierpathogener Mikroben, woraus sich breit gefächerte Anwendungsmöglichkeiten ergeben.

Die erfindungsgemäßen Verbindungen können auch in Form ihrer Säureadditionssalze verwendet werden.
Diese Säureadditionssalze können nach den üblichen Salzbildungsverfahren, zum Beispiel durch Lösen einer Verbindung der Formel I in einem geeigneten Lösungsmittel und Hinzufügen der Säure erhalten werden.

Zur Bildung der Säureadditionssalze kommen sowohl anorganische wie auch organische Säuren in Frage. Als Beispiele seien genannt: Halogenwasserstoffsäuren, wie zum Beispiel die Chlorwasserstoffsäure und die Bromwasserstoffsäure, ferner Phosphorsäure, Schwefelsäure, Salpetersäure, mono- und bifunktionelle Carbonsäuren und Hydroxycarbonsäure, wie zum Beispiel Essigsäure, Maleinsäure, Oxalsäure, Bernsteinsäure, Fumarsäure, Weinsäure, Zitronensäure, Salicylsäure, Milchsäure sowie Sulfonsäuren, wie zum Beispiel p-Toluolsulfonsäure und 1,5-Naphthalindisulfonsäure.

Als Verbindungen mit herausragender biozider und wuchsregulierender Wirkung sind insbesondere zu nennen



**Postanschrift: Schering Aktiengesellschaft, Postfach 65 03 11, D-1000 Berlin 65** · Für Besucher Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 · Berliner Commerzbank AG, Berlin, Konto-Nr. 108700600 Bankleitzahl 100 400 00 Berliner Handels- und Frankfurter Bank, Berlin Konto-Nr. 70045224, Bankleitzahl 100 202 00 Deutsche Bank Berlin AG, Konto-Nr. 2415008 Bankleitzahl 100 700 00 Postscheckamt Berlin West Konto-Nr. 1175-101 Bankleitzahl 100 100 10

E-1-(4-Chlorphenyl)-4-methoxy-4-methyl-2-(1,2,4-triazol-1-yl)-1-penten-3-ol,
E-1-(4-Fluorphenyl)-4-methoxy-4-methyl-2-(1,2,4-triazol-1-yl)-1-penten-3-ol und
E-4-Methoxy-4-methyl-1-(4-methylphenyl)-2-(1,2,4-triazol-1-yl)-1-penten-3-ol.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Zweckmäßig werden die gekennzeichnetan Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Netz-, Haft-, Emulgier- und/oder Dispergierhilfsmitteln angewandt.

Geeignete flüssige Trägerstoffe sind zum Beispiel Wasser, aliphatische und aromatische Kohlenwasserstoffe, wie Benzol, Toluol, Xylol, Cyclohexanon, Isophoron, Dimethylsulfoxyd, Dimethylformamid, weiterhin Mineralölfraktionen und Pflanzenöle.

Als feste Trägerstoffe eignen sich Mineralerden, zum Beispiel Bentonite, Silicagel, Talkum, Kaolin, Attapulgit, Kalkstein, Kieselsäure und pflanzliche Produkte, zum Beispiel Mehle.

An oberflächenaktiven Stoffen sind zu nennen: zum Beispiel Calciumligninsulfonat, Polyoxyäthylenalkylphenyl-äther, Naphthalinsulfonsäuren und deren Salze, Phenolsulfonsäuren und deren Salze, Formaldehydkondensate, Fettalkoholsulfate

-5-

sowie substituierte Benzolsulfonsäuren und deren Salze.

Sofern die Wirkstoffe zur Saatgutbeizung Verwendung finden sollen, können auch Farbstoffe zugemischt werden, um dem gebeizten Saatgut eine deutlich sichtbare Färbung zu geben.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen,, zum Beispiel mit Wasser als Träger in Spritzbrühmengen von etwa 100 bis 1000 Liter/ha. Eine Anwendung der Mittel im sogenannten Low-Volume- oder Ultra-Low-Volume-Verfahren ist ebenso möglich wie ihrerApplication in Form von sogenannten Mikrogranulaten.

Zur Herstellung der Zubereitungen werden zum Beispiel die folgenden Bestandteile eingesetzt:

a) 80 Gewichtsprozent Wirkstoff
   15 Gewichtsprozent Kaolin
   5 Gewichtsprozent oberflächenaktive Stoffe auf der Basis einer Mischung des Calciumsalzes der Ligninsulfonsäure mit Alkylphenolpolyglykoläthern

b) 45 Gewichtsprozent Wirkstoff
   5 Gewichtsprozent Natriumaluminiumsilikat
   15 Gewichtsprozent Cetylpolyglykoläther mit 8 Mol Äthylenoxid
   2 Gewichtsprozent Spindelöl
   10 Gewichtsprozent Polyäthylenglykol
   23 Teile Wasser

-6-

c) 20 Gewichtsprozent Wirkstoff
35 Gewichtsprozent Montmorillonit
35 Gewichtsprozent Kieselsäure
8 Gewichtsprozent Zellpech
2 Gewichtsprozent Natriumsalz des N-Methyl-N-oleyltaurins

d) 20 Gewichtsprozent Wirkstoff
15 Gewichtsprozent Cyclohexanon
60 Gewichtsprozent Xylol
5 Gewichtsprozent Mischung von Nonylphenylpolyoxyäthylen oder Calciumdodecylbenzolsulfonat.

Die neuen erfindungsgemäßen Verbindungen lassen sich zum Beispiel herstellen, indem man Verbindungen der allgemeinen Formel

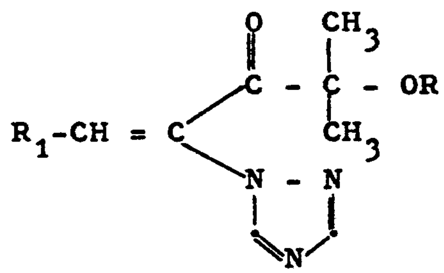

II

in Lösungsmitteln mit Metallhydriden oder Metallhydridkomplexen zum gewünschten Verfahrensprodukt reduziert.

Die E-Triazolyl-pentone der allgemeinen Formel II kann man direkt mit Hilfe geeigneter Metallhydride oder Metallhydridkomplexe in einem geeigneten Lösungsmittel reduzieren. Für diese Reduktion eignen sich Lithiumaluminiumhydrid oder Natriumborhydrid in einem geeigneten Lösungsmittel. Bei Verwendung dieser Metallhydridkomplexe können je nach Reaktionsführung wechselnde Mengen ungewünschter Nebenprodukte entstehen.



Postanschrift: Schering Aktiengesellschaft, Postfach 65 03 11, D-1000 Berlin 65 · Fur Besucher Berlin-Wedding, Mullerstraße 170-178 Telegramme: Scheringchemie
Vorstand Dr Herbert Asmis Dr Christian Bruhn, Dr Heinz Hannse, Horst Kramp, Dr Klaus Pohle, Dr Horst Witzel Vorsitzender des Aufsichtsrats: Hans-Jürgen Ha
Sitz der Gesellschaft Berlin und Bergkamen Handelsregister AG Charlottenburg 93 HRB 283 und AG Kamen HRB 0061 Berliner Commerzbank AG, Berlin, Kon
108700600, Bankleitzahl 100 400 00 Berliner Handels- und Frankfurter Bank Berlin, Konto-Nr 70045224, Bankleitzahl 100 202 00 Deutsche Bank Berlin AG, Kon
2415008, Bankleitzahl 100 700 00 Postscheckamt Berlin West Konto-Nr. 1175-101, Bankleitzahl 100 100 10

Die Bildung dieser Nebenprodukte kann überraschenderweise vermieden werden, indem man erfindungsgemäß elektrophile Reduktionsmittel in geeigneten Lösungsmitteln einsetzt. Als solche Mittel sind beispielsweise zu nennen: Aluminiumhydrid in Tetrahydrofuran oder Äther, Zinkborhydrid in Dimethoxyäthan und insbesondere Diisobutylaluminiumhydrid in Toluol, Hexan und anderen inerten Lösungsmitteln. Durch diese Verfahrenstechnik wird eine regioselektive Reduktion der Ketogruppe ermöglicht.

Die als Ausgangsprodukte benötigten E-Triazolyl-pentenone lassen sich zweckmäßigerweise aus den entsprechenden Z-Isomeren durch photochemische Umlagerung herstellen.

Dazu bestrahlt man das Z-Isomere in einem geeigneten Lösungsmittel mit Strahlen aus UV- oder Xenonlampen oder Sonnenstrahlen. Als Lösungsmittel eignen sich Alkohole wie Methanol, Äthanol oder Propanol, Äther wie Diäthyläther, Tetrahydrofuran oder Dioxan, Ketone, wie Aceton oder Methylisobutylketon, und Kohlenwasserstoffe, wie Hexan, Cyclohexan Petroläther, Benzol, Toluol oder Xylol. Die Photoisomerisierung erfolgt bevorzugt bei Temperaturen von $0^{o}$ bis $100^{o}C$ und kann auch unter Zusatz von Sensibilisatoren, wie Acetophenon, Propiophenon oder Benzophenon durchgeführt werden. Nach der Isomerisierung wird das entstandene E-Isomere durch Umkristallisieren oder Säulenchromatographie gereinigt

Die Herstellung der als Ausgangsprodukte benötigten E-Triazolyl-pentenone der allgemeinen Formel II erfolgt unter Verwendung der an sich bekannten entsprechenden Triazolyl-Butanone der allgemeinen Formel

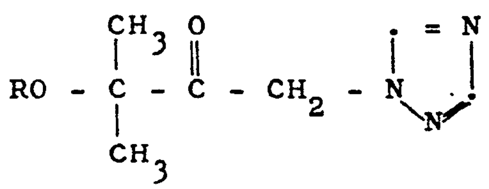 III ,



Postanschrift: Schering Aktiengesellschaft, Postfach 65 03 11, D-1000 Berlin 65 · Für Besucher: Berlin-Wedding, Müllerstraße 170-178 · Telegramme: Scheringchem
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Sitz der Gesellschaft: Berlin und Bergkamen · Handelsregister: AG Charlottenburg 93 HRB 283 und AG Kamen HRB 0061 · Berliner Commerzbank AG, Berlin, K 108700600 Bankleitzahl 100 400 00 · Berliner Handels- und Frankfurter Bank, Berlin Konto-Nr 70045224, Bankleitzahl 100 202 00 · Deutsche Bank Berlin AG, K 2415008 Bankleitzahl 100 700 00 · Postscheckamt Berlin West Konto-Nr 1175-101, Bankleitzahl 100 100 10

die mit Verbindungen der allgemeinen Formel

$$R_1 - CHO \qquad IV$$

gegebenenfalls gelöst in einem Lösungsmittel und in Gegenwart eines Katalysators kondensiert und die Reaktionsprodukte gegebenenfalls säulenchromatographisch in die gewünschten E-Isomere getrennt werden.

Im allgemeinen setzt man dabei 1 Mol des Butanons mit 1 bis 2 Mol des Aldehyds gegebenenfalls unter Anwendung eines geeigneten Lösungsmittels und in Gegenwart eines Katalysators um. Katalysatoren können beispielsweise sein: Alkali- oder Erdalkalimetallhydroxide, wie Natrium-, Kalium-,oder Calciumhydroxid, Alkalimetallalkoholate, wie Natriummethylat, Natriumäthylat oder Kaliummethylat, Carbonate, wie Natrium- oder Kaliumcarbonat, Acetate, wie Natrium- oder Kaliumacetat, sekundäre Amine, wie Diäthylamin, Dipropylamin, Pyrrolidin, Piperidin oder Morpholin, und tertiäre Amine, wie Triäthylamin, Tributylamin, Pyridin, Picolin oder Dimethylanilin. Der Katalysator gelangt in einer Menge von 0,001 bis 0,5 Mol (en) zum Einsatz. Verwendbare Lösungsmittel sind beispielsweise Alkohole, wie Methanol oder Äthanol, aromatische Kohlenwasserstoffe, wie Benzol, Toluol oder Xylol, Äther, wie Diäthyläther, Tetrahydrofuran oder Dioxan, Wasser und Mischungen hiervon. Die Umsetzung erfolgt zweckmäßigerweise im Bereich von 0°C bis zum Kochpunkt des jeweiligen Lösungsmittels.

Wenn als Katalysator ein Acetat, zum Beispiel Natrium- oder Kaliumacetat, ein Carbonat, zum Beispiel Natrium- oder Kaliumcarbonat oder ein tertiäres Amin verwendet wird, kann als Reaktionslösungsmittel Eisessig oder Essigsäureanhydrid verwendet werden.

Die Reaktionsprodukte können als Z, E-Isomerengemisch oder auch als jeweils reine Z oder E-Komponente anfallen, wobei im ersteren Fall eine säulenchromatographische Trennung un-



Postanschrift: Schering Aktiengesellschaft, Postfach 65 03 11, D-1000 Berlin 65 · Fur Besucher Berlin-Wedding, Mullerstraße 170-178 Telegramme Scheringchemie
Vorstand Dr Herbert Asmis Dr Christian Bruhn, Dr Heinz Hannse Horst Kramp, Dr. Klaus Pohle, Dr Horst Witzel Vorsitzender des Aufsichtsrats: Hans-Jürge
Sitz der Gesellschaft Berlin und Bergkamen Handelsregister AG Charlottenburg 93 HRB 283 und AG Kamen HRB 0061 · Berliner Commerzbank AG, Berlin 108700600 Bankleitzahl 100 400 00 Berliner Handels- und Frankfurter Bank, Berlin, Konto-Nr 70045224, Bankleitzahl 100 202 00 · Deutsche Bank Berlin AG 2415008, Bankleitzahl 100 700 00 Postscheckamt Berlin West Konto-Nr 1175-101 Bankleitzahl 100 100 10

ter Isolierung der gewünschten E-Isomere erfolgen muß. Wahlweise kann das Z, E-Isomerengemisch auch zunächst reduziert und anschließend säulenchromatographisch getrennt werden.

Die folgenden Beispiele erläutern die Herstellung der erfindungsgemäßen Verbindungen.



Postanschrift: Schering Aktiengesellschaft, Postfach 65 03 11, D-1000 Berlin 65 · Fur Besucher: Berlin-Wedding, Müllerstraße 170-178 · Telegramme: Scheringchemie Be
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Ham
Sitz der Gesellschaft: Berlin und Bergkamen · Handelsregister: AG Charlottenburg 93 HRB 283 und AG Kamen HRB 0061 · Berliner Commerzbank AG, Berlin, Konto 108700600, Bankleitzahl 100 400 00 · Berliner Handels- und Frankfurter Bank, Berlin, Konto-Nr 70045224, Bankleitzahl 100 202 00 · Deutsche Bank Berlin AG, Konto 2415008, Bankleitzahl 100 700 00 · Postscheckamt Berlin West, Konto-Nr 11 75-101, Bankleitzahl 100 100 10

BEISPIEL 1

E-1-(4-Chlorphenyl)-4-methoxy-4-methyl-2-(1,2,4-triazol-1-yl)-1-penten-3-ol

1,53 g E-1-(4-Chlorphenyl)-4-methoxy-4-methyl-2-(1,2,4-triazol-1-yl)-1-penten-3-on werden in 15 ml Tetrahydrofuran gelöst und auf -60°C gebracht. Unter Stickstoff werden 6 ml Diisobutylaluminiumhydrid in Toluol (ca. 1,2 m Lösung) zugetropft, wobei gerührt wird. Die Reaktion ist exotherm! Man läßt auf Raumtemperatur kommen, gibt 2 Tropfen Methanol und anschließend 15 ml 10%ige Salzsäure zu. Man rührt gut durch, gibt in einen Scheidetrichter und schüttelt mit 2 mal 50 ml Essigester aus. Nach Trocknen der organischen Phase mit $MgSO_4$ wird abfiltriert und nach Einrotieren im Vakuum bleiben 1,48 g festes Produkt vom Fp. 139°C zurück. Dieses wird aus Isopropanol umkristallisiert.

Ausbeute: 1,02 g = 70% der Theorie
Fp.: 146°C

In analoger Weise lassen sich die folgenden erfindungsgemäßen Verbindungen herstellen:



Postanschrift: Schering Aktiengesellschaft, Postfach 65 03 11, D-1000 Berlin 65 · Fur Besucher Berlin-Wedding, Mullerstraße 170-178 Telegramme: Scheringchemie
Vorstand Dr Herbert Asmis Dr Christian Bruhn Dr Heinz Hannse Horst Kramp Dr Klaus Pohle Dr Horst Witzel · Vorsitzender des Aufsichtsrats: Hans-Jürgen Ha
Sitz der Gesellschaft Berlin und Bergkamen Handelsregister AG Charlottenburg 93 HRB 283 und AG Kamen HRB 0061 Berliner Commerzbank AG, Berlin, Kor
108700600, Bankleitzahl 100 400 00 Berliner Handels- und Frankfurter Bank, Berlin, Konto-Nr. 70045224, Bankleitzahl 100 202 00 Deutsche Bank Berlin AG, Kor
2415008, Bankleitzahl 100 700 00 Postscheckamt Berlin West, Konto-Nr. 11 75-101, Bankleitzahl 100 100 10

| Beispiel Nr. | Name der Verbindungen | Physikalische Konstante |
|---|---|---|
| 2 | E-1-(2,4-Dichlorphenyl)-4-methoxy-4-methyl-2-(1,2,4-triazol-1-yl)-1-penten-3-ol | Fp.: 155-160°C |
| 3 | E-1-(4-Fluorphenyl)-4-methoxy-4-methyl-2-(1,2,4-triazol-1-yl)-1-penten-3-ol | Fp.: 157°C |
| 4 | E-4-Methoxy-4-methyl-1-(4-methylphenyl)-2-(1,2,4-triazol-1-yl)-1-penten-3-ol | Fp.: 98°C |
| 5 | E-4-Äthoxy-1-(4-fluorphenyl)-4-methyl-2-(1,2,4-triazol-1-yl)-1-penten-3-ol | Fp.: 102°C |
| 6 | E-1-(4-Bromphenyl)-4-methoxy-4-methyl-2-(1,2,4-triazol-1-yl)-1-penten-3-ol | Fp.: 135-137°C |
| 7 | E-4-Äthoxy-1-(2-fluorphenyl)-4-methyl-2-(1,2,4-triazol-1-yl)-1-penten-3-ol | Fp.: 98-101°C |
| 8 | E-4-Äthoxy-1-(4-bromphenyl)-4-methyl-2-(1,2,4-triazol-1-yl)-1-penten-3-ol | Fp.: 95°C |
| 9 | E-4-Äthoxy-1-(4-chlorphenyl)-4-methyl-2-(1,2,4-triazol-1-yl)-1-penten-3-ol | Fp.: 80°C |
| 10 | E-4-Äthoxy-4-methyl-1-(4-methylphenyl)-2-(1,2,4-triazol-1-yl)-1-penten-3-ol | $n_D^{20}$: 1,5370 |
| 11 | E-1-(2-Chlorphenyl)-4-methoxy-4-methyl-2-(1,2,4-triazol-1-yl)-1-penten-3-ol | Fp.: 162-166°C |

Die erfindungsgemäßen Verbindungen lösen sich schlecht in Wasser und mehr oder weniger gut in polaren organischen Lösungsmitteln, wie weitere Alkohole, zum Beispiel Methanol, Äthanol u.a., sowie Essigsäureäthylester, Diäthyläther, Diisopropyläther, Acetonitril und N,N-Dimethylformamid.
Sie lösen sich gut in organischen Lösungsmitteln, wie zum Beispiel Chloroform, Methylenchlorid, Toluol und Xylol.

-12-

Postanschrift: Schering Aktiengesellschaft, Postfach 65 03 11, D-1000 Berlin 65 · Fur Besucher Berlin-Wedding, Mullerstraße 170-178 Telegramme: Scheringchemie Berli
Vorstand Dr. Herbert Asmis Dr. Christian Bruhn Dr. Heinz Hannse Horst Kramp. Dr. Klaus Pohle. Dr. Horst Witzel · Vorsitzender des Aufsichtsrats: Hans-Jürgen Hamar
Sitz der Gesellschaft Berlin und Bergkamen Handelsregister AG Charlottenburg 93 HRB 283 und AG Kamen HRB 0061 Berliner Commerzbank AG, Berlin, Konto-N
108700600 Bankleitzahl 100 400 00 Berliner Handels- und Frankfurter Bank, Berlin Konto-Nr 70045224, Bankleitzahl 100 202 00 · Deutsche Bank Berlin AG, Konto-N
2415008, Bankleitzahl 100 700 00 Postscheckamt Berlin West, Konto-Nr 1175-101, Bankleitzahl 100 100 10

Die folgenden Beispiele erläutern die Herstellung der Ausgangsprodukte.

## BEISPIEL 12

Z-1-(4-Chlorphenyl)-4-methoxy-4-methyl-2-(1,2,4-triazol-1-yl)-1-penten-3-on

Zu einer Lösung von 36 g (0,196 Mol) 3-Methoxy-3-methyl-1-(1,2,4-triazol-1-yl)-2-butanon in 260 ml Toluol werden 29,66 g (0,211 Mol) 4-Chlorbenzyldehyd, 1,7 ml Piperidin und 5,6 ml Eisessig gegeben. Man kocht 5 Stunden unter Rühren am Wasserabscheider (Badtemperatur 150°C). Nach Abkühlung auf Raumtemperatur wird die Reaktionslösung mit 200 ml Essigester verdünnt und anschließend einmal mit 200 ml $H_2O$ und zweimal je 200 ml 10%iger Natriumdisulfitlösung, dann noch zweimal mit je 200 ml $H_2O$ gewaschen. Man trocknet über $MgSO_4$, filtriert und rotiert im Vakuum (20 Torr, 50°C) ein. Es bleiben ca. 60 g dunkelroten, halbkristallinen Öls zurück, das sich aus Diisopropyläther umkristallisieren läßt. Man erhält 34,2 g (57 % der Theorie) blaßgelber Kristalle vom Fp. 104-106°C.



Postanschrift: Schering Aktiengesellschaft, Postfach 65 03 11, D-1000 Berlin 65 · Fur Besucher Berlin-Wedding, Müllerstraße 170-178 Telegramme: Scheringchemie B
Vorstand Dr Herbert Asmis Dr Christian Bruhn, Dr Heinz Hannse Horst Kramp. Dr Klaus Pohle. Dr. Horst Witzel · Vorsitzender des Aufsichtsrats. Hans-Jürgen Han
Sitz der Gesellschaft Berlin und Bergkamen Handelsregister AG Charlottenburg 93 HRB 283 und AG Kamen HRB 0061 Berliner Commerzbank AG, Berlin, Konto
108700600. Bankleitzahl 100 400 00 Berliner Handels- und Frankfurter Bank Berlin. Konto-Nr 70045224. Bankleitzahl 100 202 00 · Deutsche Bank Berlin AG, Konto
2415008 Bankleitzahl 100 700 00 Postscheckamt Berlin West. Konto-Nr 11 75-101. Bankleitzahl 100 100 10

BEISPIEL 13

E-1-(4-Chlorphenyl)-4-methoxy-4-methyl-2-(1,2,4-triazol-1-yl)-1-penten-3-on

339,3 g (1,109 Mol) Z-1-(4-Chlorphenyl)-4-methoxy-4-methyl-2-(1,2,4-triazol-1-yl)-1-penten-3-on werden in 3 l Aceton gelöst und in einem Falling Film Reaktor 21 Stunden mit einer Quecksilberlampe (700 W., 254 nm) bestrahlt. Die Lösung enthält jetzt ein Z,E-Gemisch. Man rotiert zur Trockne ein und versetzt den halbkristallinen Rückstand mit 1 l Pentan. Unter Eisbadkühlung verreibt man den Rückstand mit dem Pentan und saugt anschließend das Festprodukt ab, das noch Z-Isomere enthält. 318 g des Produktes werden in 3 l Diisopropyläther unter Erhitzen gelöst, die ungelösten Flokken werden abfiltriert. Man läßt über Nacht bei -5°C kristallisieren. Die nun erhaltenen 242 g werden wieder aus 3 l Diisopropyläther umkristallisiert. Man läßt auf Raumtemperatur kommen und saugt die auskristallisierte Substanz sofort ab.

Ausbeute: 115,7 g = 34 % der Theorie
Fp.: 118-119°C

Die folgenden Ausführungsbeispiele dienen zur Erläuterung der Anwendungsmöglichkeiten der erfindungsgemäßen Verbindungen, die in Form der oben angeführten Zubereitungen erfolgte.



Postanschrift: Schering Aktiengesellschaft, Postfach 65 03 11, D-1000 Berlin 65 · Fur Besucher Berlin-Wedding, Mullerstraße 170-178 · Telegramme: Scheringchemie Be
Vorstand Dr Herbert Asmis Dr Christian Bruhn Dr Heinz Hannse Horst Kramp. Dr. Klaus Pohle. Dr. Horst Witzel Vorsitzender des Aufsichtsrats: Hans-Jürgen Hama Sitz der Gesellschaft Berlin und Bergkamen Handelsregister AG Charlottenburg 93 HRB 283 und AG Kamen HRB 0061 · Berliner Commerzbank AG. Berlin, Konto-108700600 Bankleitzahl 100 400 00 Berliner Handels- und Frankfurter Bank. Berlin. Konto-Nr. 70045224. Bankleitzahl 100 202 00 Deutsche Bank Berlin AG. Konto-2415008 Bankleitzahl 100 700 00 Postscheckamt Berlin West. Konto-Nr 11 75-101. Bankleitzahl 100 100 10

BEISPIEL 14

Wuchsreduktion bei verschiedenen Kulturpflanzen

Mais, Reis, Soja, Weizen und Zuckerrübe wurden im Vorauflaufverfahren (VA) und im Nachauflaufverfahren (NA) mit wäßrigen Emulsionen der zu prüfenden Verbindungen in Aufwandmengen von 0,5 kg Wirkstoff/ha behandelt und kultiviert.

12 Tage nach der Behandlung wurde das oberirdische Pflanzenwachstum bewertet: Die Stengel der Zweikeimblättrigen wurden bis zum Vegetationspunkt gemessen. Bei den Einkeimblättrigen wurde die Länge des Halms im Vorauflaufversuch bis zum Ausbiegen der ersten Blattspreite, im Nachauflaufversuch bis zum Ausbiegen der zweiten Blattspreite gemessen.

Die so ermittelten Daten wurden in Relation zur Kontrolle gesetzt und die prozentuale Wuchshemmung ermittelt.

Die Ergebnisse machen deutlich, daß die erfindungsgemäßen Verbindungen entweder bei mehreren Kulturen in einem der beiden Anwendungsverfahren dem Vergleichsmittel überlegen sind oder mindestens bei einer Kultur oder einer Anwendungsweise eine stärkere Wirkung zeigen, darüber hinaus aber auch noch in den anderen Kulturen zu einer Wuchshemmung führen.



Postanschrift: Schering Aktiengesellschaft, Postfach 65 03 11, D-1000 Berlin 65 · Fur Besucher Berlin-Wedding, Mullerstraße 170-178 · Telegramme: Scheringchemie I
Vorstand Dr Herbert Asmis Dr Christian Bruhn Dr Heinz Hannse Horst Kramp Dr Klaus Pohle. Dr Horst Witzel Vorsitzender des Aufsichtsrats: Hans-Jürgen Ha
Sitz der Gesellschaft Berlin und Bergkamen Handelsregister AG Charlottenburg 93 HRB 283 und AG Kamen HRB 0061 Berliner Commerzbank AG. Berlin, Kon
108700600. Bankleitzahl 100 400 00 Berliner Handels- und Frankfurter Bank. Berlin. Konto-Nr 70045224. Bankleitzahl 100 202 00 Deutsche Bank Berlin AG. Kon
2415008. Bankleitzahl 100 700 00 Postscheckamt Berlin West Konto-Nr 11 75-101. Bankleitzahl 100 100 10

| Erfindungsgemäße Verbindungen | Wuchshemmung in % | | | | | | | | | |
|---|---|---|---|---|---|---|---|---|---|---|
| | Mais | | Reis | | Soja | | Weizen | | Zuckerrübe | |
| | VA | NA | VA | NA | VA | NA | VA | NA | VA | NA |
| E-1-(4-Chlorphenyl)-4-methoxy-4-methyl-2-(1,2,4-triazol-1-yl)-1-penten-3-ol | 30 | 45 | 42 | 33 | 89 | 65 | 60 | 40 | 62 | 0 |
| E-4-Äthoxy-1-(4-bromphenyl)-4-methyl-2-(1,2,4-triazol-1-yl)-1-penten-3-ol | 10 | 34 | 0 | 21 | 78 | 63 | 20 | 28 | 75 | 12 |
| E-4-Äthoxy-1-(4-chlorphenyl)-4-methyl-2-(1,2,4-triazol-1-yl)-1-penten-3-ol | 0 | 28 | 25 | 33 | 81 | 60 | 30 | 32 | 62 | 12 |
| E-4-Äthoxy-4-methyl-1-(4-methylphenyl)-2-(1,2,4-triazol-1-yl)-1-penten-3-ol | 20 | 38 | 0 | 8 | 72 | 62 | 30 | 9 | 37 | 0 |
| Vergleichsmittel gemäß DE-OS 30 10 560 | | | | | | | | | | |
| E-1-(4-Chlorphenyl)-4,4-dimethyl-2-(1,2,4-triazol-1-yl)-1-penten-3-ol | 10 | 34 | 25 | 50 | 83 | 62 | 40 | 40 | 62 | 0 |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen H
Sitz der Gesellschaft: Berlin und Bergkamen · Handelsregister: AG Charlottenburg 93 HRB 283 und AG Kamen HRB 0061 · Berliner Commerzbank AG, Berlin, Ko
108700600, Bankleitzahl 100 400 00 · Berliner Handels- und Frankfurter Bank, Berlin, Konto-Nr. 70045224, Bankleitzahl 100 202 00 · Deutsche Bank Berlin AG, Ko
2415008, Bankleitzahl 100 700 00 · Postscheckamt Berlin West, Konto-Nr. 1175-101, Bankleitzahl 100 100 10
Formular Nr. 34 91 4

BEISPIEL 15

Wuchsreduktion und Streßtoleranz bei verschiedenen Kulturpflanzen

Baumwolle, Mais, Reis, Soja und Weizen wurden wie für Beispiel 14 beschrieben behandelt und ausgewertet.

Nach der Längenmessung, also am 12. Tag, wurden die Pflanzen dieses Versuchs nicht mehr gegossen. Nach zwei Tagen wurde der Grad der Welke bei Baumwolle, Mais und Soja in 4 Stufen festgehalten:

0 = Pflanze vollturgeszent
1 = Anzeichen von Welke
2 = Blätter welk
3 = Blätter stark welk
4 = Blätter trocken, papierartig

Es zeigt sich, daß die erfindungsgemäßen Verbindungen die Streßtoleranz gegenüber dem Vergleichsmittel und der Kontrolle deutlich steigern, überdies sind auch die wuchshemmenden Effekte bei vielen Behandlungen stärker als die des Vergleichsmittels.



Postanschrift: Schering Aktiengesellschaft, Postfach 65 03 11, D-1000 Berlin 65 · Fur Besucher Berlin-Wedding, Müllerstraße 170-178 · Telegramme: Scheringc

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürg
Sitz der Gesellschaft: Berlin und Bergkamen · Handelsregister: AG Charlottenburg 93 HRB 283 und AG Kamen HRB 0061 · Berliner Commerzbank AG, Berl 108700600, Bankleitzahl 100 400 00 · Berliner Handels- und Frankfurter Bank, Berlin, Konto-Nr. 70045224, Bankleitzahl 100 202 00 · Deutsche Bank Berlin A 2415008, Bankleitzahl 100 700 00 · Postscheckamt Berlin West, Konto-Nr 1175-101, Bankleitzahl 100 100 10

| Erfindungsgemäße Verbindungen | Baumwolle VA WH | Baumwolle VA T | Baumwolle NA WH | Baumwolle NA T | Mais VA WH | Mais VA T | Mais NA WH | Mais NA T | Reis VA WH | Reis NA WH |
|---|---|---|---|---|---|---|---|---|---|---|
| E-1-(2,4-Dichlorphenyl-4-methoxy-4-methyl-2-(1,2,4-triazol-1-yl)-1-penten-3-ol | 0 | 2 | 29 | 1 | 0 | 0 | 38 | 0 | 0 | 0 |
| E-1-(4-Fluorphenyl)-4-methoxy-4-methyl-2-(1,2,4-triazol-1-yl)-1-penten-3-ol | 21 | 0 | 21 | 0 | 0 | 0 | 31 | 2 | 0 | 0 |
| E-4-Methoxy-4-methyl-1-(4-methylphenyl)-2-(1,2,4-triazol-1-yl)-1-penten-3-ol | 29 | 0 | 36 | 0 | 0 | 0 | 24 | 0 | 0 | 17 |
| E-4-Äthoxy-1-(4-fluorphenyl)-4-methyl-2-(1,2,4-triazol-1-yl)-1-penten-3-ol | 11 | 0 | 21 | 0 | 0 | 0 | 24 | 2 | 17 | 8 |
| E-1-(4-Bromphenyl)-4-methoxy-4-methyl-2-(1,2,4-triazol-1-yl)-1-penten-3-ol | 36 | 0 | 43 | 0 | 0 | 0 | 38 | 0 | 17 | 29 |
| E-4-Äthoxy-1-(2-fluorphenyl)-4-methyl-2-(1,2,4-triazol-1-yl)-1-penten-3-ol | 0 | 0 | 0 | 0 | 0 | 0 | 10 | 0 | 0 | 0 |
| E-1-(2-Chlorphenyl)-4-methoxy-4-methyl-2-(1,2,4-triazol-1-yl)-1-penten-3-ol | 0 | 2 | 28 | 0 | 0 | 0 | 17 | 2 | 0 | 0 |
| **Vergleichsmittel** | | | | | | | | | | |
| 2-Chloräthyltrimethylammonium-chlorid (Chlormequatchloride) | 12 | 2 | 32 | 2 | 0 | 2 | 28 | 4 | 0 | 0 |
| **Kontrolle** | 0 | 3 | 0 | 3 | 0 | 2 | 0 | 4 | 0 | 0 |

T = Toleranz (Boniturwerte)
WH = Wuchshemmung in %

NA = Nachauflauf
VA = Vorauflauf



Postanschrift: Schering Aktiengesellschaft, Postfach 65 03 11, D-1000 Berlin 65 · Für Besucher Berlin-Wedding, Müllerstraße 170-178 Telegramme: Scheringchemie
Vorstand Dr. Herbert Asmis Dr. Christian Bruhn Dr. Heinz Hannse Horst Kramp Dr. Klaus Pohle, Dr. Horst Witzel · Vorsitzender des Aufsichtsrats: Hans-Jürgen H
Sitz der Gesellschaft Berlin und Bergkamen Handelsregister AG Charlottenburg 93 HRB 283 und AG Kamen HRB 0061 Berliner Commerzbank AG, Berlin, Ko
108700600 Bankleitzahl 100 400 00 Berliner Handels- und Frankfurter Bank, Berlin, Konto-Nr 70045224, Bankleitzahl 100 202 00 · Deutsche Bank Berlin AG, Ko
2415008, Bankleitzahl 100 700 00 Postscheckamt Berlin West, Konto-Nr 11 75-101 Bankleitzahl 100 100 10

| Erfindungsgemäße Verbindungen | Soja VA WH | Soja VA T | Soja NA WH | Soja NA T | Weizen VA WH | Weizen NA WH | Zuckerrübe VA WH | Zuckerrübe NA WH |
|---|---|---|---|---|---|---|---|---|
| E-1-(2,4-Dichlorphenyl-4-methoxy-4-methyl-2-(1,2,4-triazol-1-yl)-1-penten-3-ol | 0 | 0 | 27 | 2 | 10 | 27 | 0 | 0 |
| E-1-(4-Fluorphenyl)-4-methoxy-4-methyl-2-(1,2,4-triazol-1-yl)-1-penten-3-ol | 39 | 0 | 50 | 0 | 40 | 18 | 50 | 0 |
| E-4-Methoxy-4-methyl-1-(4-methylphenyl)-2-(1,2,4-triazol-1-yl)-1-penten-3-ol | 78 | 0 | 67 | 0 | 30 | 27 | 37 | 0 |
| E-4-Äthoxy-1-(4-fluorphenyl)-4-methyl-2-(1,2,4-triazol-1-yl)-1-penten-3-ol | 0 | 0 | 57 | 0 | 20 | 23 | 37 | 0 |
| E-1-(4-Bromphenyl)-4-methoxy-4-methyl-2-(1,2,4-triazol-1-yl)-1-penten-3-ol | 0 | 0 | 57 | 2 | 24 | 36 | 37 | 0 |
| E-4-Äthoxy-1-(2-fluorphenyl)-4-methyl-2-(1,2,4-triazol-1-yl)-1-penten-3-ol | 22 | 0 | 0 | 2 | 0 | 5 | 0 | 0 |
| E-1-(2-Chlorphenyl)-4-methoxy-4-methyl-2-(1,2,4-triazol-1-yl)-1-penten-3-ol | 0 | 2 | 0 | 4 | 10 | 23 | 12 | 38 |
| **Vergleichsmittel** | | | | | | | | |
| 2-Chloräthyltrimethylammonium-chlorid (Chlormequatchloride) | 0 | 2 | 0 | 4 | 20 | 23 | 12 | 0 |
| **Kontrolle** | 0 | 2 | 0 | 4 | 0 | 0 | 0 | 0 |

T = Toleranz (Boniturwerte)
WH = Wuchshemmung in %

NA = Nachauflauf
VA = Vorauflauf

-19-

BEISPIEL 16

Wuchsreduktion bei Mais

Auf Mais wurden die in der nachfolgenden Tabelle aufgeführten Verbindungen als wäßrige Emulsionen in Aufwandmengen von 0,1 und 0,5 kg Wirkstoff/ha im Vorauflaufverfahren appliziert.

Nach 12 Tagen wurde die Länge der Stengel bis zum Ausbiegen des ersten Blattes (wie in Beispiel 14) gemessen und in Relation zur unbehandelten Kontrolle gesetzt. Danach wurde die prozentuale Wuchshemmung ermittelt.

| Erfindungsgemäße Verbindung | Wuchshemmung in % 0,5 kg/ha | 0,1 kg/ha |
|---|---|---|
| E-1-(4-Chlorphenyl)-4-methoxy-4-methyl-2-(1,2,4-triazol-1-yl)-1-penten-3-ol | 56 | 33 |
| Vergleichsmittel gemäß DE-OS 30 10 560 | | |
| 1-(4-Chlorphenyl)-4,4-dimethyl-2-(1,2,4-triazol-1-yl)-1-penten-3-ol | 22 | 11 |

Den Ergebnissen ist zu entnehmen, daß die erfindungsgemäße Verbindung gegenüber dem Vergleichsmittel eine mehr als doppelt so starke Wirkung entfaltet. Die behandelten Pflanzen sind gestaucht und dunkelgrün.



Postanschrift: Schering Aktiengesellschaft, Postfach 65 03 11, D-1000 Berlin 65 · Für Besucher Berlin-Wedding, Müllerstraße 170-178 Telegramme: Scheringchemie
Vorstand Dr Herbert Asmis Dr Christian Bruhn Dr Heinz Hannse Horst Kramp, Dr Klaus Pohle, Dr Horst Witzel · Vorsitzender des Aufsichtsrats: Hans-Jürgen Ha
Sitz der Gesellschaft Berlin und Bergkamen Handelsregister AG Charlottenburg 93 HRB 283 und AG Kamen HRB 0061 · Berliner Commerzbank AG, Berlin, Kor
108700600 Bankleitzahl 100 400 00 Berliner Handels- und Frankfurter Bank, Berlin, Konto-Nr 70045224, Bankleitzahl 100 202 00 Deutsche Bank Berlin AG, Kor
2415008 Bankleitzahl 100 700 00 Postscheckamt Berlin West, Konto-Nr 11 75-101, Bankleitzahl 100 100 10

BEISPIEL 17

Wirkung prophylaktischer Blattbehandlung gegen Erysiphe graminis an Gerstenpflanzen im Gewächshaus

Junge Gerstenpflanzen wurden mit 0,025 % Wirkstoffkonzentration tropfnaß gespritzt. Nach Antrocknen des Spritzbelages wurden die behandelten sowie unbehandelten Pflanzen inokuliert, indem man befallene Pflanzen über die Versuchspflanzen strich, so daß die Konidiosporen von Erysiphe graminis trocken übertragen wurden. Danach wurden die Versuchspflanzen im Gewächshaus bei 20 bis 22°C inkubiert. Nach einer Woche wurde der prozentuale Befall der Blätter notiert. Aus den Zahlen errechnete man die Fungizidwirkung wie folgt:

$$100 - \frac{100 \cdot \text{Befall in Behandelt}}{\text{Befall in Unbehandelt}} = \%\ \text{Wirkung}$$

Die Verbindungen lagen als 20 %ige Formulierungen vor.



Postanschrift: Schering Aktiengesellschaft, Postfach 65 03 11, D-1000 Berlin 65 · Fur Besucher Berlin-Wedding, Mullerstraße 170-178 Telegramme Scheringchemie
Vorstand Dr Herbert Asmis, Dr Christian Bruhn, Dr Heinz Hannse Horst Kramp, Dr. Klaus Ponte, Dr. Horst Witzel Vorsitzender des Aufsichtsrats: Hans-Jurgen H
Sitz der Gesellschaft Berlin und Bergkamen Handelsregister AG Charlottenburg 93 HRB 283 und AG Kamen HRB 0061 Berliner Commerzbank AG, Berlin, Ko 108700600. Bankleitzahl 100 400 00 Berliner Handels- und Frankfurter Bank, Berlin, Konto-Nr 70045224, Bankleitzahl 100 202 00 Deutsche Bank Berlin AG, Ko 2415008, Bankleitzahl 100 700 00 Postscheckamt Berlin West Konto-Nr 1175-101, Bankleitzahl 100 100 10

TABELLE

% Wirkung der prophylaktischen Spritzbehandlung

| Erfindungsgemäße Verbindungen | Erysiphe graminis |
|---|---|
| E-1-(4-Chlorphenyl)-4-methoxy-4-methyl-2-(1,2,4-triazol-1-yl)-1-penten-3-ol | 100 |
| E-1-(2,4-Dichlorphenyl)-4-methoxy-4-methyl-2-(1,2,4-triazol-1-yl)-1-penten-3-ol | 95 |
| E-1-(4-Fluorphenyl)-4-methoxy-4-methyl-2-(1,2,4-triazol-1-yl)-1-penten-3-ol | 100 |
| E-4-Methoxy-4-methyl-1-(4-methylphenyl)-2-(1,2,4-triazol-1-yl)-1-penten-3-ol | 100 |
| E-4-Äthoxy-1-(4-fluorphenyl)-4-methyl-2-(1,2,4-triazol-1-yl)-1-penten-3-ol | 100 |
| E-1-(4-Bromphenyl)-4-methoxy-4-methyl-2-(1,2,4-triazol-1-yl)-1-penten-3-ol | 100 |
| E-4-Äthoxy-1-(2-fluorphenyl)-4-methyl-2-(1,2,4-triazol-1-yl)-1-penten-3-ol | 100 |
| E-4-Äthoxy-1-(4-bromphenyl)-4-methyl-2-(1,2,4-triazol-1-yl)-1-penten-3-ol | 100 |
| E-4-Äthoxy-1-(4-chlorphenyl)-4-methyl-2-(1,2,4-triazol-1-yl)-1-penten-3-ol | 100 |
| E-4-Äthoxy-4-methyl-1-(4-methylphenyl)-2-(1,2,4-triazol-1-yl)-1-penten-3-ol | 100 |
| E-1-(2-Chlorphenyl)-4-methoxy-4-methyl-2-(1,2,4-triazol-1-yl)-1-penten-3-ol | 100 |

-22-

BEISPIEL 18

Wirkung prophylaktischer Blattbehandlung gegen Puccinia hordei (Zwergrost) an Gerstenpflanzen im Gewächshaus

Junge Gerstenpflanzen wurden mit 0,025 % Wirkstoffkonzentration tropfnaß gespritzt, Nach Antrocknen des Spritzbelages wurden die behandelten sowie die unbehandelten Pflanzen trocken inokuliert, indem man zwergrost-befallene Pflanzen über die Versuchspflanzen strich. Anschließend wurden die Versuchspflanzen bei 15 °C und hoher Luftfeuchtigkeit im Gewächshaus inkubiert.

Nach anderthalb Wochen wurde der prozentuale Anteil befallener Blattfläche notiert. Die Fungizidwirkung errechnete man wie folgt:

$$100 - \frac{100 \cdot \text{Befall in Behandelt}}{\text{Befall in Unbehandelt}} = \% \text{ Wirkung}$$

Die Verbindungen lagen als 20 %ige Formulierungen vor.



Postanschrift: Schering Aktiengesellschaft, Postfach 65 03 11, D-1000 Berlin 65 · Fur Besucher Berlin-Wedding, Mullerstraße 170-178 Telegramme Scheringchemie Be
Vorstand Dr Herbert Asmis Dr Christian Bruhn Dr Heinz Hannse Horst Kramp Dr Klaus Pohle, Dr Horst Witzel Vorsitzender des Aufsichtsrats: Hans-Jurgen Ham
Sitz der Gesellschaft Berlin und Bergkamen Handelsregister AG Charlottenburg 93 HRB 283 und AG Kamen HRB 0061 Berliner Commerzbank AG, Berlin Konto 108700600 Bankleitzahl 100 400 00 Berliner Handels- und Frankfurter Bank Berlin, Konto-Nr 70045224 Bankleitzahl 100 202 00 Deutsche Bank Berlin AG, Konto 2415008, Bankleitzahl 100 700 00 Postscheckamt Berlin West Konto-Nr 1175-101 Bankleitzahl 100 100 10

TABELLE

% Wirkung der prophylaktischen Spritzbehandlung

| Erfindungsgemäße Verbindungen | Puccinia hordei |
|---|---|
| E-1-(4-Chlorphenyl)-4-methoxy-4-methyl-2-(1,2,4-triazol-1-yl)-1-penten-3-ol | 100 |
| E-1-(2,4-Dichlorphenyl)-4-methoxy-4-methyl-2-(1,2,4-triazol-1-yl)-1-penten-3-ol | 80 |
| E-1-(4-Fluorphenyl)-4-methoxy-4-methyl-2-(1,2,4-triazol-1-yl)-1-penten-3-ol | 100 |
| E-4-Methoxy-4-methyl-1-(4-methylphenyl)-2-(1,2,4-triazol-1-yl)-1-penten-3-ol | 80 |
| E-4-Äthoxy-1-(4-fluorphenyl)-4-methyl-2-(1,2,4-triazol-1-yl)-1-penten-3-ol | 95 |
| E-1-(4-Bromphenyl)-4-methoxy-4-methyl-2-(1,2,4-triazol-1-yl)-1-penten-3-ol | 100 |
| E-4-Äthoxy-1-(2-fluorphenyl)-4-methyl-2-(1,2,4-triazol-1-yl)-1-penten-3-ol | 100 |
| E-4-Äthoxy-1-(4-bromphenyl)-4-methyl-2-(1,2,4-triazol-1-yl)-1-penten-3-ol | 100 |
| E-4-Äthoxy-1-(4-chlorphenyl)-4-methyl-2-(1,2,4-triazol-1-yl)-1-penten-3-ol | 100 |
| E-4-Äthoxy-4-methyl-1-(4-methylphenyl)-2-(1,2,4-triazol-1-yl)-1-penten-3-ol | 100 |
| E-1-(2-Chlorphenyl)-4-methoxy-4-methyl-2-(1,2,4-triazol-1-yl)-1-penten-3-ol | 100 |

**Postanschrift: Schering Aktiengesellschaft, Postfach 65 03 11, D-1000 Berlin 65** · Für Besucher: Berlin-Wedding, Müllerstraße 170-178 · Telegramme: Scheringche

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürge
Sitz der Gesellschaft: Berlin und Bergkamen · Handelsregister: AG Charlottenburg 93 HRB 283 und AG Kamen HRB 0061 · Berliner Commerzbank AG, Berlin, 108700600, Bankleitzahl 100 400 00 · Berliner Handels- und Frankfurter Bank, Berlin, Konto-Nr. 70045224, Bankleitzahl 100 202 00 · Deutsche Bank Berlin AG, 2415008, Bankleitzahl 100 700 00 · Postscheckamt Berlin West, Konto-Nr. 1175-101, Bankleitzahl 100 100 10

BEISPIEL 19

Saatgutbehandlung gegen Tilletia caries im Freilandversuch

Mit 5 g Sporen vom Steinbranderreger Tilletia caries wurde jeweils 1 kg Weizensaatgut künstlich kontaminiert und unbehandelt bzw. nach Behandlung mit dem in der Tabelle angegebenen Verbindungen im Freiland ausgesät. Die zu prüfenden Verbindungen wurden als Emulsionskonzentrate in 1 l Wasser pro 100 kg Saatgut eingesetzt. Nach etwa 4 Monaten (für Sommerweizen) wurden die kranken Ähren gezählt und daraus die Wirkung berechnet

Wirkstoffaufwand 20 g/ 100 kg Saatgut

| Erfindungsgemäße Verbindung | % Wirkung Tilletia caries an Sommerweizen |
|---|---|
| E-1-(4-Chlorphenyl)-4-methoxy-4-methyl-2-(1,2,4-triazol-1-yl)-1-penten-3-ol | 100 |
| Vergleichsmittel gemäß DE-OS 30 10 560 | |
| E-1-(4-Chlorphenyl)-4,4-dimethyl-2-(1,2,4-triazol-1-yl)-1-penten-3-ol | 100 |

Das Vergleichsmittel erwies sich als stark phytotoxisch indem der Weizen gegenüber dem erfindungsgemäß behandelten um 60 % geschädigt wurde.

-25-

BEISPIEL 20

Saatgutbehandlung gegen Helminthosporium gramineum der Sommergerste im Freiland

Gerstensaatgut mit natürlichem Befall von Helminthosporium gramineum (Erreger der Streifenkrankheit) wurde unbehandelt bzw. nach Behandlung mit den in der Tabelle angegebenen Verbindungen im Freiland ausgesät. Die zu prüfenden Verbindungen wurden als Emulsionskonzentrate in 1 l Wasser pro 100 kg Saatgut eingesetzt. Nach 3 Monaten wurden die befallenen Pflanzen gezählt und daraus die Wirkung berechnet.

Wirkstoffaufwand 20 g/ 100 kg Saatgut

| Erfindungsgemäße Verbindung | % Wirkung Helminthosporium gramineu an Sommer - Gerste |
|---|---|
| E-1-(4-Chlorphenyl)-4-methoxy-4-methyl-2-(1,2,4-triazol-1-yl)-1-penten-3-ol | 92 |
| Vergleichsmittel gemäß DE-OS 30 10 560 | |
| E-1-(4-Chlorphenyl)-4,4-dimethyl-2-(1,2,4-triazol-1-yl)-1-penten-3-ol | - |

Die Gerste wurde durch das Vergleichsmittel völlig vernichtet, so daß eine Beurteilung der fungiziden Wirkung nicht möglich war.



Postanschrift: Schering Aktiengesellschaft, Postfach 65 03 11, D-1000 Berlin 65 · Fur Besucher Berlin-Wedding, Müllerstraße 170-178 · Telegramme: Scheringc
Vorstand Dr Herbert Asmis Dr Christian Bruhn, Dr Heinz Hannse, Horst Kramp, Dr Klaus Pohle, Dr. Horst Witzel · Vorsitzender des Aufsichtsrats: Hans-Jürg
Sitz der Gesellschaft Berlin und Bergkamen Handelsregister AG Charlottenburg 93 HRB 283 und AG Kamen HRB 0061 Berliner Commerzbank AG, Berli
108700600, Bankleitzahl 100 400 00 Berliner Handels- und Frankfurter Bank, Berlin, Konto-Nr 70045224, Bankleitzahl 100 202 00 Deutsche Bank Berlin AG
2415008, Bankleitzahl 100 700 00 Postscheckamt Berlin West Konto-Nr 1175-101, Bankleitzahl 100 100 10

## BEISPIEL 21

Saatgutbehandlung gegen Ustilago avenae an Hafer im Freilandversuch

Hafersaatgut wurde in Suspension der Sporen des Haferflugbrandes Ustilago avenae getaucht und in einem Vakuum-Exsikkator mehrmals dem Wechsel von Normal- und Unterdruck ausgesetzt. Nach dem Trocknen des Saatgutes wurde mit den in der Tabelle angegebenen Verbindungen behandelt, die als Emulsionskonzentrat in 1 l Wasser pro 100 kg Saatgut eingesetzt wurden. 10 Wochen nach der Aussaat des behandelten Saatgutes sowie unbehandelter infizierter Körner als Kontrolle wurden die kranken Rispen gezählt und daraus die Wirkung berechnet.

**Wirkstoffaufwand 50 g/ 100 kg Saatgut**

| Erfindungsgemäße Verbindung | % Wirkung Ustilago avenae an Hafer |
|---|---|
| E-1-(4-Chlorphenyl)-4-methoxy-4-methyl-2-(1,2,4-triazol-1-yl)-1-penten-3-ol | 100 |
| Vergleichsmittel gemäß DE-OS 30 10 560 | |
| 1-(4-Chlorphenyl)-4,4-dimethyl-2-(1,2,4-triazol-1-yl)-1-penten-3-ol | 100 |

Der Hafer wurde durch das Vergleichsmittel um 30 % geschädigt

-27-

Postanschrift: Schering Aktiengesellschaft, Postfach 65 03 11, D-1000 Berlin 65 · Für Besucher Berlin-Wedding, Müllerstraße 170-178 Telegramme: Scheringchemie
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen H Sitz der Gesellschaft: Berlin und Bergkamen Handelsregister: AG Charlottenburg 93 HRB 283 und AG Kamen HRB 0061 Berliner Commerzbank AG, Berlin, Ko 108700600, Bankleitzahl 100 400 00 Berliner Handels- und Frankfurter Bank, Berlin Konto-Nr. 70045224 Bankleitzahl 100 202 00 Deutsche Bank Berlin AG, Ko 2415008 Bankleitzahl 100 700 00 Postscheckamt Berlin West Konto-Nr. 1175-101 Bankleitzahl 100 100 10

BEISPIEL 22

Wirkung prophylaktischer Blattbehandlung gegen Botrytis cinerea an Tomatensämlingen im Gewächshaus

Junge Tomatenpflanzen wurden mit 0,025 % Wirkstoff enthalten der Spritzflüssigkeit tropfnaß behandelt. Nach Antrocknen des Spritzbelages wurden die behandelten Pflanzen sowie unbehandelte Pflanzen als Kontrolle inokuliert. Hierzu wurde als Inokulat eine Suspension von Sporen (etwa 1 Million je Milliliter Fruchtsaftlösung) des Grauschimmelerregers Botrytis cinerea auf die Pflanzen gesprüht. Anschließend wurden die Pflanzen im Gewächshaus bei etwa 20°C und hoher Luftfeuchtigkeit inkubiert. Nach dem Zusammenbrechen der unbehandelten Pflanzen (=100 % Befall) wurde der Befallsgrad der behandelten Pflanzen festgestellt und die Fungizidwirkung errechnet.

Die erfindungsgemäße Verbindung E-1-(4-Chlorphenyl)-4-methoxy-4-methyl-2-(1,2,4-triazol-1-yl)-1-penten-3-ol, in 10 %iger Formulierung angewandt, schränkte den Befall um 80 % ein.

-28-

Postanschrift: Schering Aktiengesellschaft, Postfach 65 03 11, D-1000 Berlin 65 · Für Besucher Berlin-Wedding, Müllerstraße 170-178 Telegramme: Scheringchemie
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen
Sitz der Gesellschaft: Berlin und Bergkamen Handelsregister: AG Charlottenburg 93 HRB 283 und AG Kamen HRB 0061 Berliner Commerzbank AG, Berlin 108700600 Bankleitzahl 100 400 00 Berliner Handels- und Frankfurter Bank Berlin Konto-Nr. 70045224, Bankleitzahl 100 202 00 Deutsche Bank Berlin AG 2415008 Bankleitzahl 100 700 00 Postscheckamt Berlin West, Konto-Nr. 11 75-101, Bankleitzahl 100 100 10

BEISPIEL 23

Wirkung prophylaktischer und kurativer Behandlung gegen Apfelschorf (Venturia inaequalis) im Freiland

Im Wachstum befindliche Apfeltriebe wurden mit 300 ppm Wirkstoff enthaltender Spritzflüssigkeit tropfnaß behandelt. Nach dem Antrocknen des Spritzbelages wurden diese Triebe sowie weitere als unbehandelte Kontrolle bzw. für die Kurativbehandlung inokuliert. Als Inokulat wurde eine Suspension von 345 000 Sporen pro Milliliter 3 %iger Glukoselösung in Aqua dest. unter Vermeidung des Abtropfens auf die Blätter gesprüht. Die inokulierten Triebe wurden sofort in Polyäthylenbeutel eingehüllt. Nach 2 Tagen wurden die Beutel abgenommen. 1 Woche nach der Inokulation wurde die Kurativbehandlung an inokulierten, bisher unbehandelten Trieben ausgeführt.

3 1/2 Wochen nach der Inokulation wurde der prozentuale Anteil vom Schorf befallener Blattfläche notiert und daraus die fungizide Wirkung der Behandlung errechnet.

| Erfindungsgemäße Verbindung | % Wirkung gegen Venturia inaequalis prophylaktisch | kurativ |
|---|---|---|
| E-1-(4-Chlorphenyl)-4-methoxy-4-methyl-2-(1,2,4-triazol-1-yl)-1-penten-3-ol | 99 | 79 |
| **Vergleichsmittel** | | |
| N-(Trichlormethylthio)-phthalimid | 100 | 0 |

-29-

BEISPIEL 24

Wirkung prophylaktischer Blattbehandlung gegen Cochliobolus an Reissämlingen im Gewächshaus

Junge Reispflanzen wurden mit 250 ppm Wirkstoffkonzentration tropfnaß gespritzt. Nach Antrocknen des Spritzbelages wurden die behandelten Pflanzen sowie unbehandelte Kontrollpflanzen durch Aufsprühen einer Suspension von Sporen (etwa 250 000/ml) des Pilzes Cochliobolus miyabeanus inokuliert und bei 24 bis 26°C in feuchtegesättigter Luft im Gewächshaus inkubiert. Nach 5 Tagen schätzte man den prozentualen Anteil der vom Pilz zerstörten Blattfläche.

| Erfindungsgemäße Verbindungen | % Wirkung gegen Cochliobolus an Reis |
|---|---|
| E-4-Äthoxy-1-(4-bromphenyl)-4-methyl-2-(1,2,4-triazol-1-yl)-1-penten-3-ol | 90 |
| E-4-Äthoxy-1-(4-chlorphenyl)-4-methyl-2-(1,2,4-triazol-1-yl)-1-penten-3-ol | 93 |
| E-4-Äthoxy-4-methyl-1-(4-methylphenyl)-2-(1,2,4-triazol-1-yl)-1-penten-3-ol | 93 |
| E-1-(2-Chlorphenyl)-4-methoxy-4-methyl-2-(1,2,4-triazol-1-yl)-1-penten-3-ol | 85 |

-30-

Postanschrift: Schering Aktiengesellschaft, Postfach 65 03 11, D-1000 Berlin 65 · Für Besucher Berlin-Wedding, Müllerstraße 170-178 Telegramme: Scheringchem
Vorstand Dr. Herbert Asmis Dr. Christian Bruhn Dr. Heinz Hannse Horst Kramp Dr. Klaus Pohle Dr. Horst Witzel · Vorsitzender des Aufsichtsrats: Hans-Jürgen
Sitz der Gesellschaft Berlin und Bergkamen Handelsregister AG Charlottenburg 93 HRB 283 und AG Kamen HRB 0061 · Berliner Commerzbank AG, Berlin, K
108700600 Bankleitzahl 100 400 00 Berliner Handels- und Frankfurter Bank, Berlin, Konto-Nr 70045224, Bankleitzahl 100 202 00 Deutsche Bank Berlin AG, K
2415008, Bankleitzahl 100 700 00 Postscheckamt Berlin West, Konto-Nr 11 75-101, Bankleitzahl 100 100 10

BEISPIEL 25

Wirkung prophylaktischer Blattbehandlung gegen Piricularia oryzae an Reissämlingen im Gewächshaus

Junge Reispflanzen wurden mit 250 ppm Wirkstoffkonzentration tropfnaß gespritzt. Nach Antrocknen des Spritzbelages wurden die behandelten Pflanzen sowie unbehandelte Kontrollpflanzen durch Aufsprühen einer Suspension von Sporen (etwa 200 000/ml) des Blattfleckenerregers Piricularia oryzae inokuliert und feucht bei +25° bis +27°C im Gewächshaus inkubiert.

Nach 5 Tagen wurde festgestellt, wieviel Prozent der Blattfläche befallen war.

| Erfindungsgemäße Verbindungen | % Wirkung gegen Piricularia an Reis |
|---|---|
| E-4-Äthoxy-1-(4-bromphenyl)-4-methyl-2-(1,2,4-triazol-1-yl)-1-penten-3-ol | 85 |
| E-4-Äthoxy-1-(4-chlorphenyl)-4-methyl-2-(1,2,4-triazol-1-yl)-1-penten-3-ol | 93 |
| E-4-Äthoxy-4-methyl-1-(4-methylphenyl)-2-(1,2,4-triazol-1-yl)-1-penten-3-ol | 100 |



Postanschrift: Schering Aktiengesellschaft, Postfach 65 03 11, D-1000 Berlin 65 · Fur Besucher: Berlin-Wedding, Mullerstraße 170-178 Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Haman
Sitz der Gesellschaft: Berlin und Bergkamen Handelsregister: AG Charlottenburg 93 HRB 283 und AG Kamen HRB 0061 Berliner Commerzbank AG, Berlin, Konto-N 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-N 2415008, Bankleitzahl 100 700 00 Postscheckamt Berlin West, Konto-Nr. 1175-101, Bankleitzahl 100 100 10

BEISPIEL 26

Wirkung prophylaktischer Blattbehandlung gegen Helminthosporium teres (=Pyrenophora teres), Netzfleckenkrankheit der Gerste im Gewächshaus

Junge Gerstenpflanzen im Stadium des ersten Blattes wurden mit 250 ppm Wirkstoff-Konzentration tropfnaß gespritzt. Nach Antrocknen der Spritzbeläge wurden die behandelten Pflanzen und unbehandelte Kontrollpflanzen mit einer Susoension der Konidiosporen von Helminthosporium teres besprüht und in einer Feuchtkammer des Gewächshauses 2 Tage bei 20 bis 22°C inkubiert. Danach wurden die Pflanzen im Gewächshaus bei 20 bis 22°C kultiviert. Eine Woche nach der Inokulation, wurde der Prozentuale Befall der Blattflächen notiert.

| Erfindungsgemäße Verbindungen | % Wirkung gegen Helminthosporium an Gerste |
|---|---|
| E-4-Äthoxy-1-(2-fluorphenyl)-4-methyl-2-(1,2,4-triazol-1-yl)-1-penten-3-ol | 100 |
| E-4-Äthoxy-1-(4-bromphenyl)-4-methyl-2-(1,2,4-triazol-1-yl)-1-penten-3-ol | 93 |
| E-4-Äthoxy-1-(4-chlorphenyl)-4-methyl-2-(1,2,4-triazol-1-yl)-1-penten-3-ol | 95 |
| E-4-Äthoxy-4-methyl-1-(4-methylphenyl)-2-(1,2,4-triazol-1-yl)-1-penten-3-ol | 99 |
| E-1-(2-Chlorphenyl)-4-methoxy-4-methyl-2-(1,2,4-triazol-1-yl)-1-penten-3-ol | 99 |

-32-

BEISPIEL 27

Wirkung prophylaktischer Blattbehandlung gegen Erysiphe cichoracearum an Kürbispflanzen im Gewächshaus.

Mit den angegebenen Wirkstoffkonzentrationen tropfnaß gespritzte Kürbispflanzen wurden nach Antrocknen des Spritzbelages durch Aufstäuben trockener Mehltausporen von Erysiphe cichoracearum inokuliert. Die Pflanzen wurden im Gewächshaus bei 24°C inkubiert. Nach einer Woche wurde die befallene Blattfläche in % von der gesamten Blattfläche geschätzt. Die Fungizidwirkung berechnete man wie folgt:

$$100 - \frac{100 \text{ . Befall in Behandelt}}{\text{Befall in Unbehandelt}} = \text{\% Wirkung}$$

Die Verbindungen lagen als 10 %ige oder 20 %ige Formulierungen vor.

| Erfindungsgemäße Verbindungen | % Wirkung mit 5ppm | 0,5 ppm |
|---|---|---|
| E-4-Äthoxy-1-(2-fluorphenyl)-4-methyl--2-(1,2,4-triazol-1-yl)-1-penten-3-ol | 100 s | 100 s |
| E-4-Äthoxy-1-(4-bromohenyl)-4-methyl--2-(1,2,4-triazol-1-yl)-1-penten-3-ol | 100 s | 100 |
| E-4-Äthoxy-1-(4-chlorphenyl)-4-methyl--2-(1,2,4-triazol-1-yl)-1-penten-3-ol | 100 s | 100 s |
| E-4-Äthoxy-4-methyl-1-(4-methylphenyl)--2-(1,2,4-triazol-1-yl)-1-penten-3-ol | 100 s | 100 |
| E-1-(2-Chlorphenyl)-4-methoxy-4-methyl--2-(1,2,4-triazol-1-yl)-1-penten-3-ol | 100 s | 97,5 |

Der Zusatz "s" bedeutet systemische Wirkung, erkennbar am Schutz des unbehandelten Zuwachses der Pflanzen.

-33-

BEISPIEL 28

Wirkung prophylaktischer Blattbehandlung gegen Uromyces appendiculatus an Bohnen im Gewächshaus.

Bohnenpflanzen im Stadium der Primärblätter wurden mit 4 ppm Wirkstoff-Konzentration tropfnaß gespritzt. Nach Antrocknen der Spritzbeläge wurden die behandelten Pflanzen sowie unbehandelte Kontrollpflanzen mit Suspension der Bohnenrostsporen (400 000 pro Milliliter) besprüht und drei Tage in feuchter Luft bei 22°C gehalten. Danach wurden die Pflanzen im Gewächshaus aufgestellt. 20 Tage nach der Inokulation wurde der prozentuale Anteil befallener Blattfläche notiert. Zugleich wurden etwaige phytotoxische Schäden notiert.

Die fungizide Wirkung wurde wie folgt berechnet:

$$100 - \frac{100 \cdot \text{Befall in Behandelt}}{\text{Befall in Unbehandelt}} = \%\ \text{Wirkung}$$

| Erfindungsgemäße Verbindung | % Wirkung mit 4 ppm |
|---|---|
| E-1-(4-Chlorphenyl)-4-methoxy-4-methyl-2-(1,2,4-triazol-1-yl)-1-penten-3-ol | 99,5 |
| Vergleichsmittel gemäß DE-OS 30 10 560 | |
| E-1-(4-Chlorphenyl)-4,4-dimethyl-2-(1,2,4-triazol-1-yl)-1-penten-3-ol | 100 * |

* Gegenüber der erfindungsgemäßen Verbindung schädigte das Vergleichsmittel die Pflanzen um 15 %.

Die Verbindungen lagen als 10 %ige Emulsionskonzentrate vor.



Postanschrift: Schering Aktiengesellschaft, Postfach 65 03 11, D-1000 Berlin 65 · Für Besucher Berlin-Wedding, Müllerstraße 170-178 Telegramme: Scheringchemie
Vorstand Dr. Herbert Asmis Dr. Christian Bruhn Dr. Heinz Hannse. Horst Kramp. Dr. Klaus Pohle. Dr. Horst Witzel Vorsitzender des Aufsichtsrats: Hans-Jürg
Sitz der Gesellschaft Berlin und Bergkamen Handelsregister AG Charlottenburg 93 HRB 283 und AG Kamen HRB 0061 Berliner Commerzbank AG, Berlin
108700600. Bankleitzahl 100 400 00 Berliner Handels- und Frankfurter Bank Berlin. Konto-Nr 70045224, Bankleitzahl 100 202 00 Deutsche Bank Berlin AG
2415008. Bankleitzahl 100 700 00 Postscheckamt Berlin West. Konto-Nr 11 75-101. Bankleitzahl 100 100 10

BEISPIEL 29

Microbizide Wirkung in vitro gegen Pseudomonas phaseolicola und Mucor spec.
Pseudomonas phaseolicola ist bekannt als Erreger der Fettfleckenkrankheit der Bohne. Mucor spec. war von erkranktem Gewebe der Sojabohne isoliert worden.

Wäßrige Wirkstoffzubereitungen wurden mit sterilisiertem flüssigem Agarnährboden (2 % Biomalz, 2 % Agar) bei etwa 45°C in der Weise vermischt, daß die Mischung 250 ppm Aktivsubstanz enthielt. Die Mischung wurde in Polystyrol-Petrischalen von 8,5 cm Durchmesser 5 mm hoch gegossen. Nach dem Erkalten des Nährbodens wurde in die Mitte der Petrischale eine Suspension von Pseudomonas phaseolicola in destilliertem Wasser mittels 5 mm großer Impf-Öse abgetupft bzw. ein 5 mm großes Impfstück mit Mucor abgesetzt. Je Behandlung und Organismus wurden 2 Schalen und von Unbehandelt 4 Schalen beimpft. Nach 5 Tagen (Mucor) bzw. nach 3 Wochen (Pseudomonas) Inkubation bei 20 bis 22°C im Dunkel wurde der Koloniedurchmesser bestimmt und nach Abzug der Inokulatgröße wie folgt zur Berechnung der Hemmwirkung herangezogen:

$$100 - \frac{\text{Durchmesser ohne Inokulat in Behandelt} \cdot 100}{\text{Durchmesser ohne Inokulat in Unbehandelt}} = \text{\% Hemmwirkung}$$

Die erfindungsgemäßen Verbindungen lagen als 10 oder 20 %ige in Wasser dispergierte Formulierungen vor.



Postanschrift: Schering Aktiengesellschaft, Postfach 65 03 11, D-1000 Berlin 65 · Für Besucher Berlin-Wedding, Müllerstraße 170-178 Telegramme Scheringchemi
Vorstand: Dr. Herbert Asmis Dr. Christian Bruhn Dr. Heinz Hannse Horst Kramp Dr. Klaus Pohle Dr. Horst Witzel Vorsitzender des Aufsichtsrats Hans-Jürgen I
Sitz der Gesellschaft Berlin und Bergkamen Handelsregister AG Charlottenburg 93 HRB 283 und AG Kamen HRB 0061 Berliner Commerzbank AG, Berlin, K
1087006600 Bankleitzahl 100 400 00 Berliner Handels- und Frankfurter Bank Berlin Konto-Nr 70045224 Bankleitzahl 100 202 00 Deutsche Bank Berlin AG, K
2415008 Bankleitzahl 100 700 00 Postscheckamt Berlin West Konto-Nr 1175-101 Bankleitzahl 100 100 10

| Erfindungsgemäße Verbindungen | % Wirkung gegen Pseudomonas 250 ppm | Mucor 250 ppm |
|---|---|---|
| E-1-(4-Chlorphenyl)-4-methoxy-4-methyl-2-(1,2,4-triazol -1-yl)-1-penten-3-ol | 97 | 89 |
| E-1-(2,4-Dichlorphenyl)-4-methoxy-4-methyl-2-(1,2,4-triazol-1-yl)-1-penten-3-ol | 90 | |
| E-1-(4-Fluorphenyl)-4-methoxy-4-methyl-2-(1,2,4-triazol-1-yl)-1-penten-3-ol | 100 | |
| E-4-Methoxy-4-methyl-1-(4-methylphenyl)-2-(1,2,4-triazol-1-yl)-1-penten-3-ol | 97 | |
| E-4-Äthoxy-1-(4-fluorphenyl)-4-methyl-2-(1,2,4-triazol-1-yl)-1-penten-3-ol | 91 | |
| E-1-(4-Bromphenyl)-4-methoxy-4-methyl-2-(1,2,4-triazol-1-yl)-1-penten-3-ol | 93 | |
| E-4-Äthoxy-1-(2-fluorphenyl)-4-methyl-2-(1,2,4-triazol-1-yl)-1-penten-3-ol | 93 | 80 |
| E-4-Äthoxy-1-(4-bromphenyl-4-methyl-2-(1,2,4-triazol-1-yl)-1-penten-3-ol | 97 | 93 |
| E-4-Äthoxy-1-(4-chlorphenyl)-4-methyl-2-(1,2,4-triazol-1-yl)-1-penten-3-ol | 100 | 81 |
| E-4-Äthoxy-4-methyl-1-(4-methylphenyl)-2-(1,2,4-triazol-1-yl)-1-penten-3-ol | 100 | |
| E-1-(2-Chlorphenyl)-4-methoxy-4-methyl-2-(1,2,4-triazol-1-yl)-1-penten-3-ol | 90 | |

-36-

Postanschrift: Schering Aktiengesellschaft, Postfach 65 03 11, D-1000 Berlin 65 · Fur Besucher Berlin-Wedding, Mullerstraße 170-178 Telegramme: Scheringchemie

Vorstand Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen H
Sitz der Gesellschaft: Berlin und Bergkamen · Handelsregister: AG Charlottenburg 93 HRB 283 und AG Kamen HRB 0061 · Berliner Commerzbank AG, Berlin, Kto
108700600 Bankleitzahl 100 400 00 · Berliner Handels- und Frankfurter Bank, Berlin, Konto-Nr. 70045224, Bankleitzahl 100 202 00 · Deutsche Bank Berlin AG, Kto
2415008, Bankleitzahl 100 700 00 · Postscheckamt Berlin West, Konto-Nr. 1175-101, Bankleitzahl 100 100 10

BEISPIEL 30

Im Gewächshaus wurden die aufgeführten Pflanzen nach dem Auflaufen mit der erfindungsgemäßen Verbindung und dem Vergleichsmittel in der Aufwandmenge von 3 kg/ha behandelt. Die Verbindungen wurden zu diesem Zweck als Suspension mit 500 Liter Wasser/ha gleichmäßig auf die Pflanzen ausgebracht. 2 Wochen nach der Behandlung wurde bonitiert nach dem Boniturschema

0 = Vernichtung der Pflanzen
1-4 = Sehr stark Inhibition der Pflanzen
5-7 = Starke Inhibition der Pflanzen
8-9 = Geringe Inhibition der Pflanzen
10 = Normales Wachstum der Pflanzen

Aus der Tabelle geht hervor, daß besonders viele Unkrautarten durch die erfindungsgemäße Verbindung vollkommen in ihrer Entwicklung inhibiert werden, so daß sie als Konkurrenz ausgeschaltet werden können.
Dieser Effekt kann zur Unkrautbekämpfung benutzt werden.
Eine gleich gute Wirkung zeigte das Vergleichsmittel nicht.

| | Erfindungsgemäße Verbindung<br>E-1-(4-Fluorphenyl)-4-methoxy-4-methyl-2-(1,2,4-triazol-1-yl)-1-penten-3-ol | Vergleichsmittel gem. DE.OS 3148742<br>1-(4-Fluorphenyl)-4-methoxy-4-methyl-2-(1,2,4-triazol-1-yl)-1-penten-3-ol |
|---|---|---|
| Helianthus sp. | 3 | 8 |
| Cucumus sp. | 4 | 8 |
| Stellaria media | 6 | 8 |
| Centaurea c. | 4 | 8 |
| Ipomea p. | 5 | 10 |
| Polygonum f. | 7 | 10 |
| Solanum sp. | 6 | 9 |
| Triticum sp. | 5 | 8 |
| Hordeum | 6 | 9 |
| Alopecurus sp | 6 | 10 |

-37-

BEISPIEL 31

Im Gewächshaus wurden die aufgeführten Pflanzen vor dem Auflaufen mit der erfindungsgemäßen Verbindung E-1-(4-Chlorphenyl)-4-methoxy-4-methyl-2-(1,2,4-triazol-1-yl)-1-penten-3-ol in der Aufwandmenge von 0,3 kg Wirkstoff/ha behandelt.

Die Verbindung wurde zu diesem Zweck als Suspension mit 500 Liter Wasser/ha gleichmäßig auf die Pflanzen ausgebracht.
2 Wochen nach der Behandlung wurde bonitiert nach dem Boniturschema

0 = Vernichtung der Pflanzen
1 - 4 = sehr starke Inhibition der Pflanzen
5 - 7 =starke Inhibition der Pflanzen
8 - 9 = geringe Inhibition der Pflanzen
10 = normales Wachstum der Pflanzen

Aus der Tabelle geht hervor, daß besonders viele Unkrautarten durch die erfindungsgemäße Verbindung in ihrer Entwicklung inhibiert werden, so daß sie als Konkurrenz ausgeschaltet werden können.
Dieser Effekt kann zur Unkrautbekämpfung genutzt werden.



Postanschrift: Schering Aktiengesellschaft, Postfach 65 03 11, D-1000 Berlin 65 · Fur Besucher. Berlin-Wedding, Mullerstraße 170-178 · Telegramme: Scheringchemie

Vorstand: Dr. Herbert Asmis. Dr. Christian Bruhn. Dr. Heinz Hannse. Horst Kramp. Dr. Klaus Pohle. Dr. Horst Witzel · Vorsitzender des Aufsichtsrats: Hans-Jurgen
Sitz der Gesellschaft. Berlin und Bergkamen · Handelsregister: AG Charlottenburg 93 HRB 283 und AG Kamen HRB 0061 · Berliner Commerzbank AG, Berlin, 108700600 Bankleitzahl 100 400 00 · Berliner Handels- und Frankfurter Bank. Berlin. Konto-Nr 70045224. Bankleitzahl 100 202 00 · Deutsche Bank Berlin AG, 2415008 Bankleitzahl 100 700 00 · Postscheckamt Berlin West. Konto-Nr 11 75-101. Bankleitzahl 100 100 10

| Kulturpflanzen | Erfinsungsgemäße Verbindung 0,3 kg Wirkstoff/ha = Boniturnote |
|---|---|
| | E-1-(4-Chlorphenyl)-4-methoxy-4-methyl-2-(1,2,4-triazol-1-yl)-1-penten-3-ol |
| Brassica | 0 |
| Allium | 6 |
| Cucumus | 5 |
| Medicago | 5 |
| Phaseolus | 2 |
| Helianthus | 3 |
| Solanum | 3 |
| Pisum | 4 |
| Stellaria m. | 0 |
| Abutilon | 1 |
| Matricaria ch. | 1 |
| Viola tr. | 0 |
| Centarea c. | 3 |
| Amaranthus r. | 1 |
| Cassia ob. | 2 |
| Chrysanthemum s. | 4 |
| Ipomea p. | 5 |
| Fagopyrum es. | 3 |
| Zea mais | 5 |
| Triticum | 5 |
| Hordeum | 5 |
| Oryzae | 5 |
| Sorghum | 5 |
| Avena f. | 5 |
| Alopecurus m. | 6 |
| Echinochloa c.g. | 4 |
| Setaria i. | 5 |
| Digitaria s. | 3 |
| Cyperus es. | 6 |
| Poa a. | 2 |

-39-

PATENTANSPRÜCHE

1. E-Triazolyl-pentenole der allgemeinen Formel

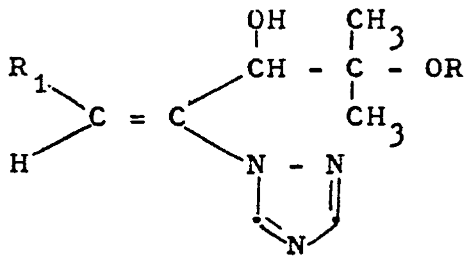

I

,

in der

R einen $C_1$-$C_{10}$-Alkylrest oder einen $C_3$-$C_8$-Alkenylrest und

$R_1$ einen nichtsubstituierten oder einen ein- oder mehrfach, gleich oder verschieden durch Halogen, $C_1$-$C_4$-Alkyl, $C_1$-$C_4$-Alkoxy, $C_1$-$C_4$-Alkylthio, Trifluormethyl oder die Nitrogruppe substituierten aromatischen Kohlenwasserstoffrest oder einen heterocyclischen Kohlenwasserstoffrest bedeuten und deren Säureadditionssalze mit anorganischen und organischen Säuren.

2. E-Triazolyl-pentenole gemäß Anspruch 1, worin

R Methyl, Äthyl, Propyl, Butyl, Pentyl, Hexyl, Heptyl, Octyl Nonyl, Decyl, Isopropyl, 2,2-Bimethylpropyl-1, 3,3-Dimethy butyl-2, 2-Buten-1-yl, 3-Methyl-2-buten-1-yl, Hexenyl, Heptenyl oder Octenyl und

$R_1$ 2-Fluorphenyl, 3-Fluorphenyl, 4-Fluorphenyl, 2-Chlorphenyl 3-Chlorphenyl, 4-Chlorphenyl, 2-Bromphenyl, 3-Bromphenyl, 4-Bromphenyl, 2-Jodphenyl, 3-Jodphenyl, 4-Jodphenyl, 2,3-Dichlorphenyl, 2,4-Dichlorphenyl, 3,4-Dichlorphenyl, 2,6-Dichlorphenyl, 2-Methylphenyl, 3-Methylphenyl, 4-Methylphenyl, 2-Äthylphenyl, 3-Äthylphenyl, 4-Äthylphenyl, 2-Propylphenyl, 3-Propylphenyl, 4-Propylphenyl, 2-Isopropylphenyl, 3-Isopropylphenyl, 4-Isopropylphenyl, 2-Butylpheny 3-Butylphenyl, 4-Butylphenyl, 2-sek.-Butylphenyl, 3-sek.-Butylphenyl, 2-tert.-Butylphenyl, 3-tert.-Butylphenyl, -4

**Postanschrift: Schering Aktiengesellschaft, Postfach 65 03 11, D-1000 Berlin 65** · Fur Besucher Berlin-Wedding, Mullerstraße 170-178 · Telegramme: Scheringchemie

Vorstand Dr Herbert Asmis, Dr Christian Bruhn, Dr Heinz Hannse, Horst Kramp, Dr Klaus Pohle, Dr Horst Witzel · Vorsitzender des Aufsichtsrats: Hans-Jurg. Sitz der Gesellschaft Berlin und Bergkamen · Handelsregister AG Charlottenburg 93 HRB 283 und AG Kamen HRB 0061 · Berliner Commerzbank AG, Berlin 108700600 Bankleitzahl 100 400 00 · Berliner Handels- und Frankfurter Bank, Berlin, Konto-Nr 70045224, Bankleitzahl 100 202 00 · Deutsche Bank Berlin AG 2415008 Bankleitzahl 100 700 00 · Postscheckamt Berlin West Konto-Nr 1175-101, Bankleitzahl 100 100 10

2-Methoxyphenyl, 3-Methoxyphenyl, 4-Methoxyphenyl, 2-Äthoxyphenyl, 3-Äthoxyphenyl, 4-Äthoxyphenyl, 2-Methylthiophenyl, 3-Methylthiophenyl, 4-Methylthiophenyl, 2-Äthylthiophenyl, 3-Äthylthiophenyl, 4-Äthylthiophenyl, 2-Trifluormethylphenyl, 3-Trifluormethylphenyl, 4-Trifluormethylphenyl, 2-Nitrophenyl, 3-Nitrophenyl, 4-Nitrophenyl, 3-Fluor-4-methoxyphenyl, 2-Chlor-5-nitrophenyl, 4-Chlor-2-fluorphenyl, 3,4,5-Trimethoxyphenyl, 5-Chlor-2-nitrophenyl, Pyridyl, Thienyl oder Furyl bedeuten.

3. E-1-(4-Chlorphenyl)-4-methoxy-4-methyl-2-(1,2,4-triazol-1-yl)-1-penten-3-ol

4. E-1-(2,4-Dichlorphenyl)-4-methoxy-4-methyl-2-(1,2,4-triazol-1-yl)-1-penten-3-ol

5. E-1-(4-Fluorphenyl)-4-methoxy-4-methyl-2-(1,2,4-triazol-1-yl)-1-penten-3-ol

6. E-4-Methoxy-4-methyl-1-(4-methylphenyl)-2-(1,2,4-triazol-1-yl)-1-penten-3-ol

7. E-4-Äthoxy-1-(4-fluorphenyl)-4-methyl-2-(1,2,4-triazol-1-yl)-1-penten-3-ol

8. E-1-(4-Bromphenyl)-4-methoxy-4-methyl-2-(1,2,4-triazol-1-yl)-1-penten-3-ol

9. E-4-Äthoxy-1-(2-fluorphenyl)-4-methyl-2-(1,2,4-triazol-1-yl)-1-penten-3-ol

10. E-4-Äthoxy-1-(4-bromphenyl)-4-methyl-2-(1,2,4-triazol-1-yl)-1-penten-3-ol

11. E-4-Äthoxy-1-(4-chlorphenyl)-4-methyl-2-(1,2,4-triazol-1-yl)-1-penten-3-ol

12. E-4-Äthoxy-4-methyl-1-(4-methylphenyl)-2-(1,2,4-triazol-1-yl)-1-penten-3-ol

13. E-1-(2-Chlorphenyl)-4-methoxy-4-methyl-2-(1,2,4-triazol-1-yl)-1-penten-3-ol

-41-

14. Verfahren zur Herstellung von E-Triazolyl-pentenolen gemäß Ansprüchen 1 bis 13, dadurch gekennzeichnet, daß man E-Triazolyl-pentenone der allgemeinen Formel

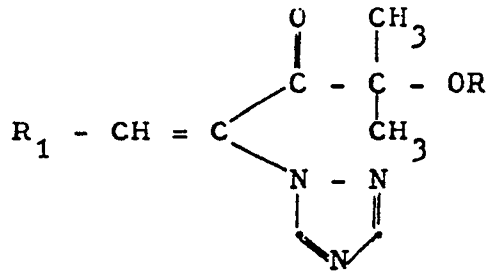

II

in Lösungsmitteln mit Metallhydriden oder Metallhydridkomplexen zum gewünschten Verfahrensprodukt reduziert.

15. Verfahren gemäß Anspruch 14, dadurch gekennzeichnet, daß elektrophile Reduktionsmittel, vorzugsweise Aluminiumhydrid, Zinkborhydrid oder Diisobutylaluminiumhydrid, verwendet werden.

16. Verfahren gemäß Anspruch 14, dadurch gekennzeichnet, daß E-Triazolyl-pentenone eingesetzt werden, die aus Z-Triazolylpentenon der allgemeinen Formel II durch photochemische Umlagerung entstanden sind.

17. Verfahren gemäß Anspruch 16, dadurch gekennzeichnet, daß die Umlagerung mittels UV-Strahlen bei Temperaturen von $0^o$ bis $100^oC$ in Lösungsmitteln gegebenenfalls unter Zusatz von Sensibilisatoren durchgeführt wird.

18. Verfahren gemäß Anspruch 14, dadurch gekennzeichnet, daß E-Triazolyl-pentenone der allgemeinen Formel II verwendet werden, die dadurch hergestellt sind, daß man Triazolylbutanone der allgemeinen Formel

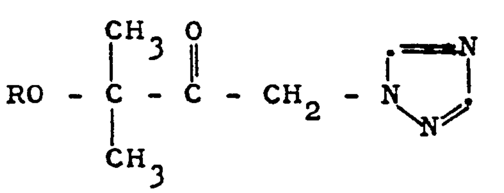

III



Postanschrift: Schering Aktiengesellschaft, Postfach 65 03 11, D-1000 Berlin 65 · Fur Besucher Berlin-Wedding, Mullerstraße 170-178 · Telegramme: Scheringchemie

Vorstand Dr Herbert Asmis Dr Christian Bruhn, Dr Heinz Hannse, Horst Kramp, Dr. Klaus Pohle, Dr. Horst Witzel Vorsitzender des Aufsichtsrats: Hans-Jürgen H
Sitz der Gesellschaft Berlin und Bergkamen Handelsregister AG Charlottenburg 93 HRB 283 und AG Kamen HRB 0061 · Berliner Commerzbank AG, Berlin, Ko
108700600 Bankleitzahl 100 400 00 Berliner Handels- und Frankfurter Bank, Berlin, Konto-Nr 70045224, Bankleitzahl 100 202 00 · Deutsche Bank Berlin AG, Ko
2415008, Bankleitzahl 100 700 00 Postscheckamt Berlin West Konto-Nr 11 75-101, Bankleitzahl 100 100 10

Mit Verbindungen der allgemeinen Formel

$$R_1 - CHO \qquad IV$$

gegebenenfalls gelöst in einem Lösungsmittel und in Gegenwart eines Katalysators kondensiert und die Reaktionsprodukte gegebenenfalls säulenchromatographisch in die gewünschten E-isomeren trennt.

19. Biozide Mittel, gekennzeichnet durch mindestens eine Verbindung gemäß den Ansprüchen 1 bis 13.

20. Biozide Mittel gemäß Anspruch 19 mit fungizider Wirkung.

21. Biozide Mittel gemäß Anspruch 19 mit bakterizider Wirkung.

22. Biozide Mittel gemäß Anspruch 19 mit herbizider Wirkung.

23. Wuchsregulatorische Mittel für Pflanzen, gekennzeichnet durch mindestens eine Verbindung gemäß den Ansprüchen 1 bis 13.

24. Mittel gemäß den Ansprüchen 19 bis 23 in Mischung mit Träger- und/oder Hilfsstoffen.

25. Mittel gemäß Ansprüchen 19 und 20 hergestellt nach Verfahren gemäß Anspruch 14



**Postanschrift: Schering Aktiengesellschaft, Postfach 65 03 11, D-1000 Berlin 65** · Fur Besucher Berlin-Wedding, Mullerstraße 170-178 Telegramme Scheringchemi

Vorstand Dr Herbert Asmis Dr Christian Bruhn Dr Heinz Hannse Horst Kramp Dr Klaus Pohle Dr. Horst Witzel Vorsitzender des Aufsichtsrats Hans-Jurgen H Sitz der Gesellschaft Berlin und Bergkamen Handelsregister AG Charlottenburg 93 HRB 283 und AG Kamen HRB 0061 Berliner Commerzbank AG, Berlin, Kc 108700600 Bankleitzahl 100 400 00 Berliner Handels- und Frankfurter Bank, Berlin Konto-Nr 70045224, Bankleitzahl 100 202 00 Deutsche Bank Berlin AG, Kc 2415008 Bankleitzahl 100 700 00 Postscheckamt Berlin West, Konto-Nr 1175-101 Bankleitzahl 100 100 10